# EUROPEAN PATENT APPLICATION

(11) **EP 4 252 804 A1**
(43) Date of publication of application: **04.10.2023**
(21) Application number: 22382289.1
(22) Date of filing: 28.03.2022
(51) Int. Cl.: A61M 11/00, A61M 15/00

(54) **NEBULIZER DEVICE**

(71) Applicant: LAINOMEDICAL S.L., 20014 Donostia-San Sebastián, Gipuzkoa (ES)
(72) Inventor: JABAT RICO, Gonzalo, Donostia-San Sebastian, Gipuzkoa (ES); PARIS HUGUET, Francesc, Donostia-San Sebastian, Gipuzkoa (ES)
(74) Representative: de Rooij, Mathieu Julien

(57) **Abstract**

The present disclosure relates to a device (10) for nebulizing a liquid composition. The device comprises a mesh (11) and an actuator for vibrating the mesh to nebulize the liquid composition. Further, the device comprises a receptacle (12) for receiving a disposable capsule (20) containing the liquid composition in a substantially vertical manner. The device also comprises a cutting element (13) for perforating a first end (21) of the disposable capsule and a channel (14) connecting the cutting element to the mesh to guide the liquid composition, and a second cutting element (201) configured to open a second end (22) of the capsule. The present disclosure also relates to methods for nebulizing a liquid composition.

## Description

### FIELD

The present disclosure relates to devices, and systems for nebulizing a liquid composition. More specifically, the present disclosure relates to systems comprising a nebulizer device and a disposable capsule. Further, the present disclosure also relates to methods for nebulizing a liquid composition.

### BACKGROUND

Nebulizer devices are used in the medical field of aerosol therapy. Nebulizers allow gaseous administration of medical and non-medical products and are generally used to treat numerous diseases, and more specifically diseases affecting the respiratory system. The nebulization, or atomization, of a liquid product allows to disperse the liquid composition into a plurality of fine droplets having diameters in the range of micrometers. These droplets are easily inhaled by the user of the nebulizer, reaching most of the respiratory system.

There are many types of nebulizer devices known in the art, such as conventional compressed air nebulizers, or ultrasonic nebulizers; the latter type using the high frequency vibration of a piezoelectric element to generate fine liquid droplets.

Some models known to date comprise a very thin membrane or mesh provided with a plurality of micrometric holes. The mesh is coupled to a piezoelectric element that vibrates at high frequency. Said vibration is transmitted to the mesh, which produces very fine nebulization of the liquid product, dispensing it to the user of the nebulizer.

Modern nebulizers are generally designed as portable devices, which should be easy and quick to use in a broad range of conditions. Almost all nebulizers on the market today require the user to obtain a container (a vial, a bottle or similar) and pour the liquid product into a reservoir in the nebulizer. This may lead to undesired liquid spills outside the dedicated opening of the nebulizer, resulting in a reduced amount of liquid to be nebulized and poor cleanliness of the nebulizer among other inconveniences. Also, it is possible for a user to, unknowingly to the user, to not provide the correct quantity, e.g. by not completely emptying a container.

Other known nebulizers are configured to receive a vessel containing a liquid product. WO2016046423 discloses a nebuliser device with a vibrating mesh, for administering medicaments, comprising a casing, a nozzle, a nebuliser and a chamber that houses a medicament, wherein said chamber housing a medicament is a disposable capsule forming an independent body that can be extracted from the casing of the device, where said disposable capsule comprises means for attaching to the nebuliser and to the casing, where said attachment means allow the disposable capsule to be coupled to the nebuliser device or uncoupled therefrom. In an example, a punch with a cutting end is provided, which when engaging the nebulizer to the disposable capsule nebulizer device pierces an external seal of the disposable capsule.

It is of interest to improve nebulizer devices and make them more reliable, robust and user friendly.

The present disclosure provides examples of devices, systems and methods that at least partially overcome some of the drawbacks of existing devices for liquid nebulization.

### SUMMARY

As a first aspect, a device for nebulizing a liquid composition is provided. The device comprises a mesh and an actuator for vibrating the mesh. The vibration of the mesh causes the nebulization of the liquid composition. Further, the device also comprises a receptacle for receiving a disposable capsule containing the liquid composition in a substantially vertical manner. The device comprises a cutting element arranged in the receptacle for perforating a first end of the disposable capsule. Additionally, the device also comprises a channel connecting the cutting element to the mesh to guide the liquid composition to the mesh due to gravity.

According to this first aspect, the device allows perforating a disposable capsule when said capsule is inserted into the dedicated receptacle. The cutting element inside the capsule stays in place during the insertion of the capsule and therefore is not prone to misalignments. Further, the device also minimizes the risk of liquid spillage and does not require the user to measure the dose to be inserted. Thus, this device results in a very user-friendly device that requires little maintenance, and which can be broadly used by patients of different ages and in several contexts.

In another aspect, a method for nebulizing a liquid composition is provided. The method comprises inserting a disposable capsule containing a liquid composition in a receptacle of a nebulizer device. The step of inserting a disposable capsule includes perforating a first end of the disposable capsule by a cutting element arranged in the receptacle of the nebulizer device. Further, the method comprises opening of the disposable capsule at a second end. Thus, gravity causes the liquid composition to pass through the cutting element and through a channel in the nebulizer device to a mesh. Additionally, the method comprises vibrating the mesh to nebulize the liquid composition.

According to this second aspect, the method allows a user to nebulize a liquid composition without carrying out any dosing or liquid handling. In fact, the user only inserts a disposable capsule in a receptacle of a nebulizer (wherein perforation occurs) and opens a second end of the disposable capsule. This equalizes pressure inside the capsule with atmospheric pressure and promotes fluid displacement into a channel of the nebulizer. The channel allows fluid communication with the mesh, which can be activated to vibrate and nebulize the composition.

The second end of the capsule is generally arranged at an opposite side of the first end. The first end may be formed e.g. by a bottom wall, and the second end may be formed e.g. by a top wall.

Throughout the present disclosure the term "capsule", may be regarded as a vessel or container comprising any liquid composition. The capsule may be manufactured using any suitable material such as a polymer composition, or a combination of materials and may have any suitable shape e.g. like a vial or bottle. In the present disclosure, a vertical direction should be understood as a direction substantially parallel to the direction of gravity. The capsules may be configured to be disposable, or "single use" or they may be configured for multiple doses (i.e. the contents of a single vial or bottle may correspond to two, three or any other specific number of treatments).

The terms "single use", or "single dose" are herein used interchangeably to denote capsules which comprise a predetermined quantity of liquid composition. The predetermined quantity corresponds to a quantity that is to be used in a single nebulization administration. After this single administration, the capsule is intended to be discarded and disposed of. The quantity contained in such a single use capsule may depend inter alia on the patient, the age of the patient, the specific medical condition of the patient, the length of time of the administration through nebulization and others. In some cases, the single use capsule may contain e.g. 5 ml or 10 ml of a liquid composition.

The term "liquid composition" is herein used to describe both liquids containing medically active ingredients in therapeutically effective amounts, and liquids which do not contain medically active ingredients, such as e.g. salted water, aromated water, physiological serums and others. The expression "therapeutically effective amount" as used herein, refers to the amount of nebulized liquid composition which is sufficient to treat the diseases to which it is addressed. The specific dose of the liquid composition or active ingredient to obtain a therapeutic benefit may vary depending on the particular circumstances of the case.

### BRIEF DESCRIPTION OF THE DRAWINGS

Non-limiting examples of the present disclosure will be described in the following, with reference to the appended drawings, in which:
Figure 1 illustrates a perspective view of a device and system according to the present disclosure in a first configuration;
Figure 2 schematically illustrates a cross-section view of a system according to another example;
Figure 3 illustrates the same perspective view of a device and system as in figure 1 in a second configuration; and
Figure 4 schematically illustrates a cross-sectional view of a system according to yet another example;
Figure 5 is a flow diagram of a method for nebulizing a liquid composition according to one example.

### DETAILED DESCRIPTION OF EXAMPLES

Reference now will be made in detail to embodiments of the present disclosure, one or more examples of which are illustrated in the drawings. Each example is provided by way of explanation only, not as a limitation. In fact, it will be apparent to those skilled in the art that various modifications and variations can be made in the present disclosure. For instance, features illustrated or described as part of one embodiment can be used with another embodiment to yield a still further embodiment. Thus, it is intended that the present disclosure covers such modifications and variations as come within the scope of the appended claims and their equivalents.

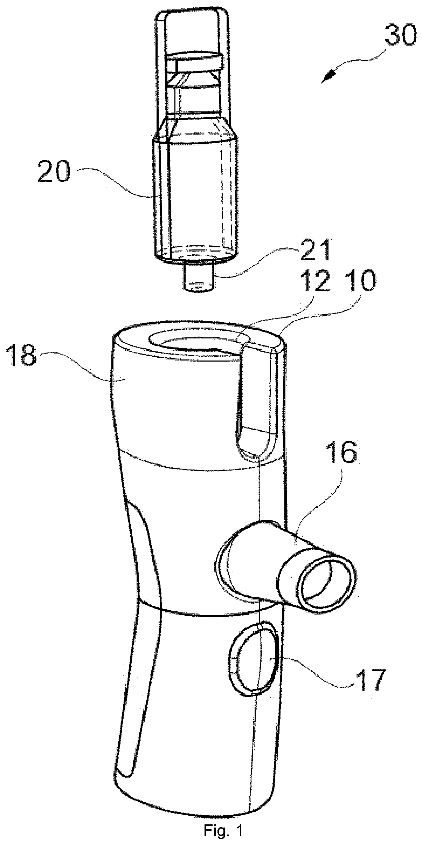

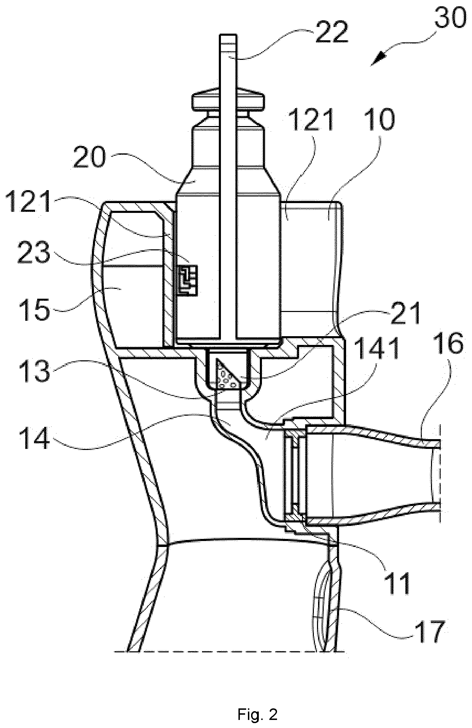

Fig. 1 illustrates a perspective view of a device according to the present disclosure. The nebulizer device 10 for nebulizing a liquid composition comprises a mesh and an actuator for vibrating the mesh to nebulize the liquid composition. Further, the nebulizer device 10 comprises a receptacle 12 for receiving a disposable capsule 20 containing the liquid composition in a substantially vertical manner. The device 10 also comprises a cutting element (not shown in figure 1) arranged in the receptacle 12 for perforating a first end 21 of the disposable capsule 20. Additionally, the device 10 comprises a channel connecting the cutting element to the mesh to guide the liquid composition to the mesh due to gravity. Note that several components of the device 10 are not shown in figure 1 since these are hidden by a casing 18 of the device 10. These and other components will be discussed in relation with figure 2.

In another aspect, figure 1 also illustrates a system 30 according to the present disclosure. The system 30 comprises the nebulizer device 10, and a disposable capsule 20. The disposable capsule 20 may be shaped and dimensioned to substantially match the receptacle 12 of the device 10. Depending on the capsule used, this may mean that the capsule can only be introduced in a single orientation (or a limited number of different orientations) into the receptacle. This may also mean that the receptacle is configured to hold the capsule 20 straight inside in the receptacle 12, i.e. with no or little deviation from a straight vertical orientation. Matching may also mean that the length of the capsule 20 and receptacle 12 are adapted to each other e.g. in such a way that the capsule 20 protrudes just beyond the edge of the receptacle so that a user can still easily access and manipulate it.

In the example illustrated in figure 1, both the disposable capsule 20 and the receptacle 12 have a substantially circular cross section along a portion of the length of the capsule, but other geometries are also possible. The disposable capsule 20 may comprise a liquid composition suitable for a single nebulizing treatment, i.e. a quantity which when emptied into the nebulizer device corresponds to a dose for a single treatment for a patient (who could be either an adult or a child).

In further examples, the disposable capsule 20 may comprise a liquid composition suitable for a single cleaning process, i.e. a quantity which when nebulized leads to a nebulizer device 10, i.e. cutting element 13, channel 14 and mesh 11 (shown e.g. in figure 2), that are substantially clean i.e. free of contaminations like dust or particles.

In some examples, the disposable capsule may comprise a liquid composition to at least partially sterilize the device, and more specifically the cutting element, the channel, and the mesh of the device 10. Liquid compositions for nebulizer sterilization may comprise acetic acid and distilled water in different ratios. When this type of disposable capsules is used, the user may operate the device as in normal use, but without inhaling the nebulized composition. In some examples, a disposable capsule with a liquid cleaning composition may be sold in a package with a plurality of capsules for normal use. E.g. a capsule with cleaning composition is included in a package with 10 or 25 (or any other suitable number) of capsules with a liquid composition for treatment of the patient.

The disposable capsules 20 may comprise different amount of liquid composition. In some cases, the disposable capsule may have a liquid volume of less than 10 ml. More specifically, the disposable capsule may have a liquid volume of less than 5 ml. Other amounts of liquid volume can be also employed. For example, liquid volumes above 10 ml or liquid volumes below 2 ml. The volume of liquid inside the disposable capsule may depend on the type of treatment and age of the user among other parameters. Further, capsules comprising a liquid composition for sterilization may comprise a liquid volume of 5ml or less.

Fig. 2 schematically illustrates a cross-section view of a system 30 according to one example. In the system 30 illustrated in figure 2 the disposable capsule 20 has already been introduced into the receptacle 12 of the device 20. Further, by doing so, the cutting element 13 has perforated a first end 21 of the disposable capsule 20. In this example, the cutting element 13 has perforated an end wall of the capsule i.e. the bottom wall of the capsule.

The first end 21 of the disposable capsule 20 may comprise a thinning of the material, i.e. the thickness of the first end 21 may be reduced in comparison with the thickness of the capsule in other locations. Said thinning may reduce the necessary pressure that a user has to apply on the disposable capsule 20 to perforate the first end 21 of the same.

Additionally, the material of the capsule 20 in the first end 21 may be more rigid than the material conforming the capsule at other locations. This increase in rigidity can limit the capacity to bend of the first end 21, and therefore may promote the perforation of the same by the cutting element 13. In other example, other frangible portions may be included to make the perforation by the cutting element easier.

In the illustrated example in figure 2, the cutting element 13 is substantially hollow and may have a substantially cylindrical portion. This allows the fluid inside the disposable capsule 20 to flow through the cutting element 13 to an internal channel 14 of the device 10. Cutting elements with other configurations are also possible. For example, the cutting element 13 could be shaped such as to leave an open area in the first end 21 of the capsule for the liquid to flow directly to the channel 14, i.e. without passing through the cutting element 13. Also in the example of figure 2, the cutting element 13 comprises a perforated wall portion to vacate nearly all liquid inside the disposable capsule 20, even the liquid below the main inlet of the cutting element 13.

Further, in the example of figure 2, the channel 14 comprises a relatively thin reservoir 141 adjacent to the mesh 11. Additionally, the channel 14 may define a liquid inlet into the reservoir 141 located in an upper portion of the reservoir 141. This configuration of the channel 14 allows the liquid composition to be stored in a reservoir 141 in contact with the mesh 11 with substantially low volume capacity. This allows maintaining most of the area of the mesh 11 in contact with the liquid composition up to a point in time when only a small portion of the liquid volume remains in the channel 14. Thus, this configuration may enhance the performance of the mesh 11, increasing the droplet throughput with a consistent distribution of droplet diameter.

Additionally, figure 2 shows that the disposable capsule 20 may comprise a bar code 23 and the device 10 may comprise a bar code reader 15. The bar code may be a linear barcode or a matrix barcode. The barcode reader 15 may read the bar code 23 and provide a signal to the user with data relative to the liquid composition inside the disposable capsule 20. In some examples, the device 10 may send the signal to a linked device, i.e. a smartphone or tablet, where the user may verify the content of the capsule among other data.

The device 10 may be configured to detect whether the capsule that has been inserted is suitable for the device, or for the patient that is registered with the device 10.

It should be clear that different identifiers (other than a barcode) may be used as well. E.g. other two-dimensional image codes such as a QR code, or other detection mechanism such as EAS (Exchange ActiveSync), RFID, NFC etc.

In some examples, the receptacle 12 may be configured to be at least partially adjustable to an external geometry of the disposable capsule 20. Thus, the internal walls 121 of the receptacle may be movable and may exert pressure on the disposable capsule 20 upon introduction of the same. In some examples, the internal walls 121 of the receptacle may comprise a resilient material such as a synthetic rubber to partially adapt the receptacle to the geometry of the capsule 20. In other examples, the internal walls 121 may be flexibly coupled to the casing 18 to provide such adaptability.

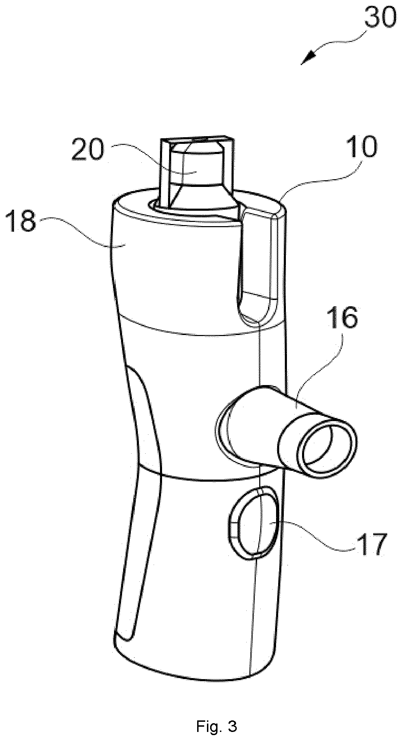

The example in figure 3 shows the device 10 and system 30 illustrated in figure 1 in a second configuration. In this configuration, a second end 22 of the disposable capsule 20 has been opened by a user. The second end 22 of the disposable capsule 20 may be a frangible end that facilitates the opening of the disposable capsule 20 by a user. Further, in other examples, the frangible end may comprise a twist cap, a tear notch, or other elements to promote the opening of the disposable capsule 20.

Additionally, figure 3 shows that a length of the receptacle 12 may be smaller than a length of the disposable capsule 20. This difference in dimensions implies that the disposable capsule 20 may be only partially introduced into the receptacle 12, with the second end 22 sticking out from the device 10 and allowing a user to easily open it. The ratio between the length of the capsule 20 inside the receptacle 12 and the overall length of the capsule 20 may be 0,3 or more, specifically 0,5 or more and more specifically 0,7 or more.

In the examples illustrated in figures 1-3, the capsule 20 is made of polyethylene, but other polymers may be also used. Such a capsule 20 may be made e.g. in a blow moulding process. More specifically, the capsule 20 may be made in a blow-fill-seal process.

Further, the disposable capsule 20 in examples may comprise more than one material, i.e. it may comprise a main body made of glass and the first and second ends made of a polymer material. Other combinations of suitable materials can be also employed.

Further, as illustrated in figures 1-3 the device 10 may comprise a nozzle 16 as an independent component from the casing 18. The nozzle 16 may be coupled to the nebulizer device 10 using different fastening mechanisms. In some examples, the coupling may be a threaded coupling. Alternatively, the coupling between the nozzle 16 and the device 10 may be performed using other types of connectors such as pressure fittings or others. The nozzle 16 may be a disposable nozzle, or a re-usable nozzle. And in further examples, the nozzle may have the shape of a fluid connection with a mask which may be attached to a patient's head e.g. for use in children.

The device 10 in this example may also comprise a start button 17 to initiate the nebulization. The start button 17 may be located in the casing 18. The start button 17 may be linked with a mechanical switch or an electronic switch configured to initiate the nebulization.

When a user or patient so indicates, a piezoelectric element arranged with the mesh may set the mesh in vibration with e.g. a predetermined frequency, for a predetermined time etc.

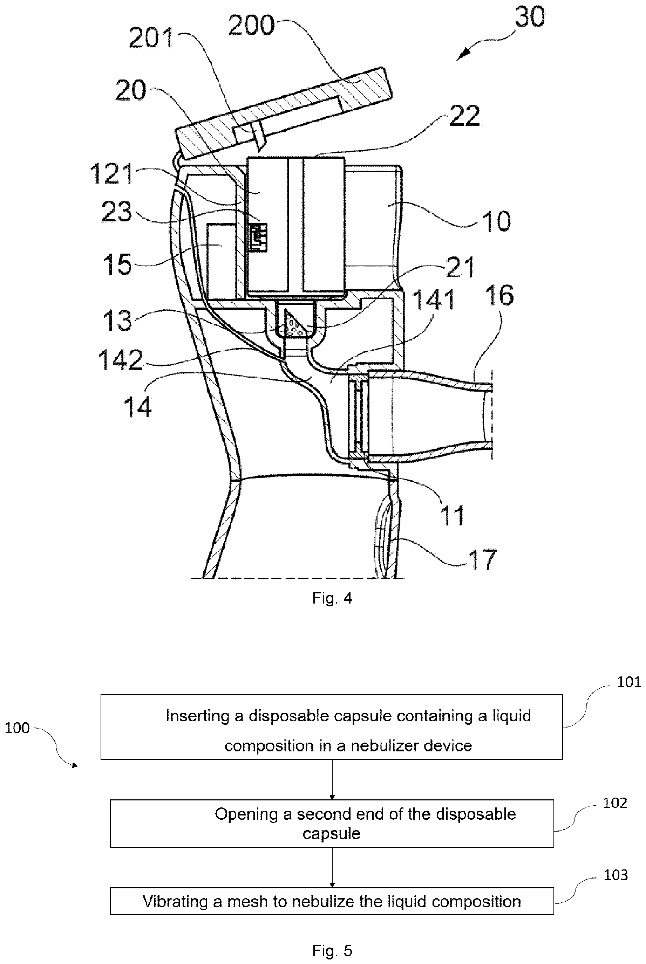

Figure 4 schematically illustrates a cross-sectional view of an example of a system 30 according to the present disclosure. The system 30 comprises a device 10 for nebulizing a liquid composition and a disposable capsule 20 comprising the liquid composition. As illustrated in figure 4, the device 10 may further comprise a lid 200 to at least partially close the receptacle of the device 10 configured to receive the capsule 20. The device 10 may comprise a rotatable connection with the lid 200, i.e. a hinged connection, but it is also possible to employ other types of connections.

In some examples, the lid 200 may comprise a second cutting element 201 configured to open the second end 22 of the capsule 20. The second cutting element 201 may be a punch, a cutting blade or others. Further, the lid 200 may be configured to be manually opened and closed by a user or it may be configured to be automatically opened and closed by a controller in the device 10.

In some examples, the lid 200 may have a recess or central depression configured to at least partially receive the second end 22 of the capsule 20. Thus, the capsule 20 may protrude from the receptacle after being inserted in the device 10 without hindering the motion of the lid 200. Additionally, the length of the second cutting element 201 may be such as to open the second end 22 of the capsule 20 when this is arranged substantially flush with a top opening of the receptacle or even if the second end 22 of the capsule 20 remains inside the receptacle, i.e. the length of the capsule 20 is smaller than the length of the receptacle.

In yet other examples, the device 10 may comprise a second cutting element within the receptacle (not shown) and which may be at least partially retractable. The second cutting element may be retracted prior inserting the capsule 20 and may be deployed after inserting the capsule 20. The deployment of the second cutting element may be triggered by the motion of the lid 200, i.e. by closing the lid 200, or it may be triggered independently from the lid, i.e. by a dedicated actuator. In this case, the second cutting element may be provided in a sidewall of the capsule at or near the second end, at a height above the liquid level in the capsule 20, which avoids or at least reduces the likeliness of spillage of the liquid composition.

As illustrated in figure 4, the device 10 (according to any of the examples disclosed herein) may comprise a conduit 142 connecting the outside of the device 10 with the channel 14. I.e. an air conduit 142 connecting the outside of the device 10 with a point of the channel between cutting element 13 and mesh 11.

The conduit 142 may include one end located in an upper section of the channel 14 and another end in a wall of the device 10e.g. at a height above the liquid level in the capsule 20. Thus, the conduit 142 may promote the displacement of the volume of air that otherwise may be trapped in the channel 14, i.e. between the mesh 11 and the first end 21 of the capsule 20 in order to facilitate emptying the entire contents of the capsule 20.

Figure 5 is a flow diagram of an example of a method 100 for nebulizing a liquid composition. In particular, figure 5 shows that the method 100 comprises, at block 101, inserting a disposable capsule 20 containing a liquid composition in a receptacle 12 of a nebulizer device 10. In the method 100, inserting 101 the disposable capsule 20 comprises perforating a first end 21 of the disposable capsule 20 by a cutting element 13 arranged in the receptacle 12 of the nebulizer device 10. Further, at block 102, the method 100 comprises opening the disposable capsule 20 at a second end 22; subsequently, gravity causes the liquid composition to pass through the cutting element 13 and through a channel 14 in the nebulizer device 10 to a mesh 11. Additionally, the method 100 also comprises, at block 103, vibrating the mesh 11 to nebulize the liquid composition.

As previously discussed, the method 100 does not require the user to pour a liquid composition into the device, nor the use of movable components to perforate the capsule 20 comprising the liquid composition. This results in a reliable and robust method that can be performed in a broad range of conditions. The use of single use capsules makes the system more user friendly requiring less maintenance, and cleaning.

In some examples, the step of inserting 101 the disposable capsule 20 comprises leaving the second end 22 of the disposable capsule 20 substantially outside the receptacle 12. This facilitates the user to reach and open the second end 22 of the same. Besides, it also facilitates the extraction of the disposable capsule 20 once the user finalizes the treatment.

Further, in some examples, the step of opening 102 the second end 22 may be performed either before or after inserting 101 the disposable capsule 20 in the receptacle 12. Thus, the user may choose to open 102 the disposable capsule 20 before inserting 101 the same if the stability of the capsule 20 inside the receptacle 12 is not guaranteed, i.e. very small capsule.

In some further examples, the step of opening 102 the second end 22 may be performed by fracturing the second end 22 of the capsule 20. Besides, other approaches such as perforating the second end 22, opening a cap in a second end 22 or others may be employed in step 102. In these examples, opening the second end comprises perforating or removing the end wall of the capsule, i.e. the top wall. In other examples that have been schematically illustrated herein, opening 102 the second end 22 may comprise opening a portion of the sidewall that is at or near the second end 22.

In any of the examples disclosed herein, the disposable capsule 20 may comprise a liquid composition for at least partially sterilizing the nebulizer or a liquid composition to be inhaled as medication.

Note that some of the technical features described in relation with the device 10 and system 30 can be included in the method 100 for nebulizing a liquid composition, and vice versa.

This written description uses examples to disclose the present teaching, including the preferred embodiments, and also to enable any person skilled in the art to practice it, including making and using any devices or systems and performing any incorporated methods. The patentable scope is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims. Aspects from the various embodiments described, as well as other known equivalents for each such aspects, can be mixed and matched by one of ordinary skill in the art to construct additional embodiments and techniques in accordance with principles of this application. If reference signs related to drawings are placed in parentheses in a claim, they are solely for attempting to increase the intelligibility of the claim, and shall not be construed as limiting the scope of the claim.

## Claims

1. A method (100) for nebulizing, comprising:
inserting (101) a disposable capsule (20) containing a liquid composition in a receptacle (12) of a nebulizer device (10), wherein inserting (101) comprises perforating a first end (21) of the disposable capsule (20) by a cutting element (13) arranged in the receptacle (12) of the nebulizer device (10); and
opening (102) of the disposable capsule (20) at a second end (22);
the liquid composition being caused by gravity to pass through the cutting element (13) and through a channel (14) in the nebulizer device (10) to a mesh (11);
vibrating (103) the mesh (11) to nebulize the liquid composition.

2. The method (100) according to claim 1, wherein inserting (101) comprises leaving the second end (22) of the disposable capsule (20) substantially outside the receptacle (12).

3. The method (100) according to any of claims 1 or 2, wherein opening (102) at the second end (22) is performed after inserting (101) the disposable capsule (20) in the receptacle (12).

4. The method (100) according to any of claims 1-3, wherein opening (102) at the second end (22) comprises at least partially fracturing the second end (22).

5. A device (10) for nebulizing a liquid composition comprising:
a mesh (11) and an actuator for vibrating the mesh (11) to nebulize the liquid composition;
a receptacle (12) for receiving a disposable capsule (20) containing the liquid composition in a substantially vertical manner,
a cutting element (13) arranged in the receptacle (12) for perforating a first end (21) of the disposable capsule (20);
a channel (14) connecting the cutting element (13) to the mesh (11) to guide the liquid composition to the mesh (11) due to gravity.

6. The device (10) according to claim 5, wherein the cutting element (13) is substantially hollow.

7. A system (30) comprising the device (10) of any of claims 5 or 6, and a disposable capsule (20), and wherein the disposable capsule optionally is made in a blow-fill-seal process.

8. The system (30) according to claim 7, wherein the disposable capsule (20) has a frangible end (22).

9. The system (30) according to any of claims 7 or 8, wherein the frangible end (22) comprises a twist cap.

10. The system (30) according to any of claims 7-9, wherein the disposable capsule (20) comprises a liquid composition suitable for a single nebulizing treatment.

11. The system (30) according to any of claims 7 - 10, wherein the disposable capsule (20) comprises a liquid composition to at least partially sterilize the cutting element (13), the channel (14) and the mesh (11) of the device (10).

12. The system (30) according to any of claims 7 - 11, wherein a length of the receptacle (12) is smaller than a length of the disposable capsule (20).

13. The system (30) according to any of claims 7 - 12, wherein the disposable capsule (20) comprises an identifier (23) and the device comprises a reader (15) for reading the identifier.

14. The system (30) according to any of claims 7 - 13, wherein the disposable capsule (20) comprises a liquid volume of less than 10 ml, and more specifically equal or less than 5 ml.

15. The system (30) according to any of claims 7 - 14, wherein the receptacle (12) of the device (10) is configured to be at least partially adjustable to an external geometry of the disposable capsule (20).
